# FASCICULE DE BREVET EUROPEEN

(11) **EP 2 151 429 B1**
(45) Date de publication et mention de la délivrance du brevet: **25.05.2011**
(21) Numéro de dépôt: 09290607.2
(22) Date de dépôt: 04.08.2009
(51) Int. Cl.: C07C 231/02, C07C 233/18, C07C 235/78

(54) **Nouveau procédé de synthèse de l'agomélatine**
Verfahren zur Herstellung von Agomelatin
Process for the preparation of agomelatin

(30) Priorité: 05.08.2008 FR 0804464
(43) Date de publication de la demande: 10.02.2010
(73) Titulaire: Les Laboratoires Servier, 92284 Suresnes Cedex (FR)
(72) Inventeur: Hardouin, Christophe, 76600 Le Havre (FR); Lecouve, Jean-Pierre, 76600 Le Havre (FR)

(56) Documents cités:
- EP-A- 0 781 764
- EP-A- 1 564 202
- D.BECKER ET AL.: "Ring opening of 3-methoxyacenaphthylene-1,2-dione..." TETRAHEDRON LETTERS, vol. 27, no. 32, 1986, pages 3775-3776, XP002518973 NLELSEVIER, AMSTERDAM

## Description

La présente invention concerne un nouveau procédé de synthèse industriel de l'agomélatine ou *N*-[2-(7-méthoxy-1-naphtyl)éthyl]acétamide de formule (I):

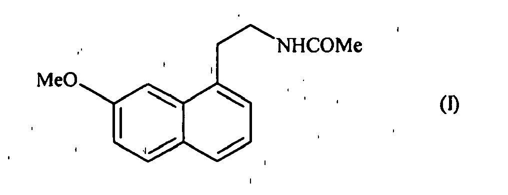

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Dans le brevet EP 1 564 202, la demanderesse a mis au point une nouvelle voie de synthèse beaucoup plus performante et industrialisable, en seulement quatre étapes à partir de la 7-méthoxy-1-tétralone, et permettant d'obtenir l'agomélatine de façon très reproductible sous une forme cristalline bien définie.

Toutefois, la recherche de nouvelles voies de synthèse, en particulier à partir de matières premières moins onéreuses que la 7-méthoxy-1-tétralone, est toujours d'actualité.

La demanderesse a poursuivi ses investigations et mis au point un nouveau procédé de synthèse de l'agomélatine à partir de la 3-méthoxyacénaphthoquinone: cette nouvelle matière première présente l'avantage d'être simple, aisément accessible en grandes quantités avec des coûts moindres. Par ailleurs, la 3-méthoxyacénaphtoquinone présente également l'avantage de posséder dans sa structure un noyau naphtalène, ce qui évite d'intégrer dans la synthèse une étape d'aromatisation, étape toujours délicate d'un point de vue industriel.

Ce nouveau procédé permet par ailleurs d'obtenir l'agomélatine de façon reproductible et sans nécessiter de purification laborieuse, avec une pureté qui est compatible avec son utilisation comme principe actif pharmaceutique.

Plus spécifiquement, la présente invention concerne un procédé de synthèse industriel du composé de formule (I) : 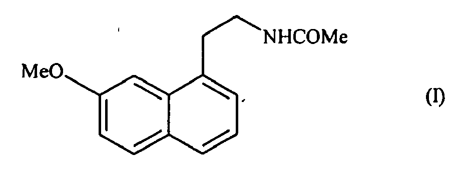 caractérisé en ce que l'on met en réaction en présence d'une base forte la 3-méthoxyacénaphtoquinone de formule (II) : 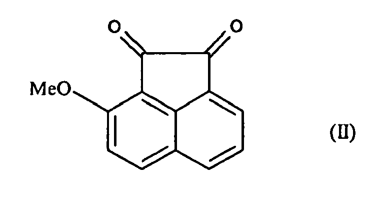 pour conduire au composé de formule (III): 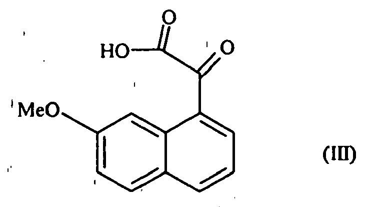 que l'on place dans des conditions d'amination pour conduire au composé de formule (IV): 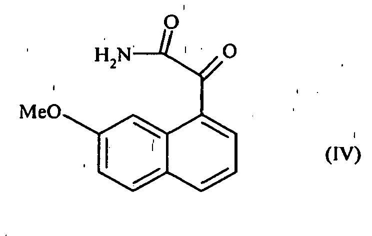 que l'on soumet à l'action d'un système réducteur pour conduire au composé de formule (V): 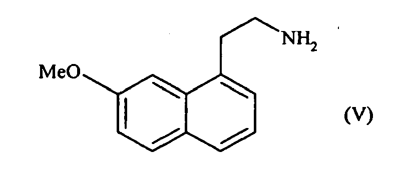 qui est successivement soumis à l'action d'acétate de sodium puis d'anhydride acétique pour conduire au composé de formule (I) que l'on isole sous la forme d'un solide.

Le composé de formule (II) est accessible à l'homme du métier par des réactions chimiques classiques et/ou décrites dans la littérature.

Avantageusement la transformation du composé de formule (II) en composé de formule (III) selon l'invention est réalisée avec NaNH₂ , ((CH₃)₃-Si)₂NLi (LiHMDS) ou ((CH₃)₃-Si)₂NNa (NaHMDS).

La réaction d'amination est réalisée préférentiellement avec NH₄Cl et l'anhydride propylphosphonique.

Le système réducteur préféré lors de la transformation du composé de formule (IV) en composé de formule (V) selon l'invention est LiAlH₄ ou le couple BH₃.THF/AlCl₃.

Ce procédé est particulièrement intéressant pour les raisons suivantes :
- il permet d'obtenir à l'échelle industrielle le composé de formule (I) avec d'excellents rendements à partir d'une matière première simple et peu onéreuse ;
- il permet d'éviter une réaction d'aromatisation puisque le noyau naphtalénique est présent dans le substrat de départ ;
- enfin, le composé de formule (I) obtenu présente de façon reproductible les caractéristiques de la forme cristalline décrite dans le brevet EP1564202.

Le composé de formule (IV) obtenu selon le procédé de l'invention est nouveau et utile en tant qu'intermédiaire de synthèse de l'agomélatine dans laquelle il est soumis à une réaction de réduction puis de couplage avec l'anhydride acétique.

Les exemples ci-dessous illustrent l'invention, mais ne la limitent en aucune façon.

### Exemple 1 : N-[2-(7-Méthoxy-1-naphtyl)éthyl]acétamide

### Stade A : Acide (7-méthoxy-1-naphtyl)(oxo)acétique

Dans un réacteur sont introduits successivement 4 mg d'éther couronne 18-crown-6 puis 230 mg de NaNH₂ à une suspension de 100 mg de 3-méthoxyacénaphtoquinone dans 1 ml de DMSO. Le mélange est agité pendant 30 minutes à température ambiante. De l'eau est ensuite ajoutée (2 ml) puis une solution de HCl 2N (3 ml). Après deux extractions avec de l'acétate d'éthyle, les solvants sont séchés sur Na₂SO₄ puis évaporés pour conduire au produit du titre sous la forme d'un solide jaune avec un rendement de 88% et une pureté chimique supérieure à 94%.
*Point de fusion : 99°C*

### Stade B : 2-(7-Méthoxy-1-naphtyl)-2-oxoacétamide

Dans un réacteur sont introduits 1 g du composé obtenu dans le Stade A dans 30 ml d'acétonitrile, puis 4,39 g d'anhydride propylphosphonique et 438 mg de NH₄Cl sont ajoutés, et en fin d'addition 3,8 ml de diisopropylamine à température ambiante. La solution est agitée 4 heures sous azote, puis les solvants sont évaporés, le résidu est repris avec une solution aqueuse saturée de NaCl, et une extraction avec de l'acétate d'éthyle est réalisée. Les solvants sont ensuite séchés sur Na₂SO₄ puis évaporés pour conduire au produit du titre sous la forme d'un solide orange avec un rendement de 80% et une pureté chimique de 90%.
*Point de fusion: 112*°C

### Stade C: 2-(7-Méthoxy-1-naphtyl)éthanamine

Dans un réacteur sont introduits 480 mg du composé obtenu dans le Stade B en solution dans le THF (20 ml), puis 2 éq de AlCl₃, et enfin lentement 6 éq de la solution BH₃.THF et le milieu réactionnel est agité pendant 2,5 heures. De l'eau est ensuite ajoutée (12 ml), puis 25 ml d'une solution de soude 1N avec 800 mg de soude solide et trois extractions au méthyltertiobutyléther (20 ml) sont réalisées. Les solvants sont ensuite séchés sur Na₂SO₄ puis évaporés pour conduire au produit du titre sous la forme d'une huile jaune avec un rendement de 80% et une pureté chimique de 95%.

### Stade D : N-[2-(7-Méthoxy-1-naphtyl)éthyl]acétamide

Dans un réacteur sont introduits 5 g du composé obtenu au stade C et 2 g d'acétate de sodium dans de l'éthanol. Le milieu est agité puis 2,3 g d'anhydride acétique sont additionnés, le milieu réactionnel est porté à reflux et 20 ml d'eau sont ajoutés. La réaction est laissée revenir à l'ambiante et le précipité obtenu est filtré, lavé par un mélange éthanol/eau 35/65 pour conduire au produit du titre avec un rendement de 80% et une pureté chimique supérieure à 99%.
*Point de fusion : 108°C*

### Exemple 2 : Détermination de la forme cristalline du composé N-[2-(7-methoxy-1-naphtyl)éthyl]acétamide obtenu dans l'Exemple 1

L'enregistrement des données a été effectué sur le diffractomètre haute résolution D8 de Bruker AXS avec les paramètres suivants : un domaine angulaire 3°-90° en 20, un pas de 0,01° et 30 s par pas. La poudre de *N*-[2-(7-méthoxy-1-naphtyl)éthyl]acélamide obtenue dans l'Exemple 1 a été déposée sur un support pour un montage en transmission. La source de rayons X est un tube au cuivre (λCuK_{α1}= 1,54056 Å). Le montage' comporte un monochromateur avant (cristal de Ge(111)) et un détecteur solide résolu en énergie (MXP-D1, Moxtec-SEPH).

Le composé est bien cristallisé : la largeur des raies à mi-hauteur est de l'ordre de 0,07° en 2θ. Les paramètres suivants ont ainsi été déterminés :
- maille cristalline monoclinique,
- paramètres de maille : a = 20,0903 Å, b = 9,3194 Å, c =15,4796 Å, β = 108,667°
- groupe d'espace : P2₁/n
- nombre de molécules dans la maille : 8
- volume de la maille:Vₘₐᵢₗₗₑ = 2746,742 Å³
- densité : d = 1,13 g/cm³.

### Exemple 3 : Détermination de la forme cristalline du composé N-[2-(7-méthoxy-1-naphtyl)éthyl]acétamide obtenu dans l'Exemple 1 par diagramme de diffraction X sur poudre

La forme cristalline du composé obtenu dans l'Exemple 1 est caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **Angle 2 theta (°)** | **Distance d inter-reticutaire (Å)** | **Intensité (%)** |
|---|---|---|
| 9,26 | 9,544 | 23 |
| 10,50 | 8,419 | 13 |
| 15,34 | 5,771 | 24 |
| 17,15 | 5,165 | 100 |

## Revendications

1. Procédé de synthèse industrielle du composé de formule (I) 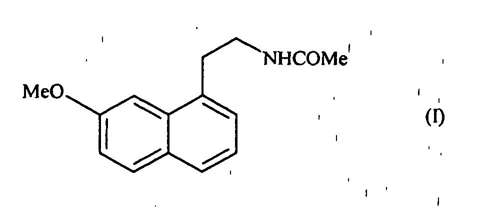 **caractérisé en ce que** l'on met en réaction en présence d'une base forte la 3-méthoxyacénaphtoquinone de formule (II): 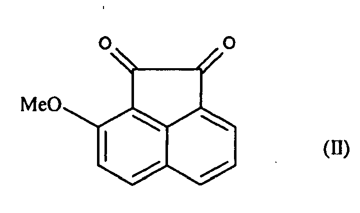 pour conduire au composé de formule (III) : 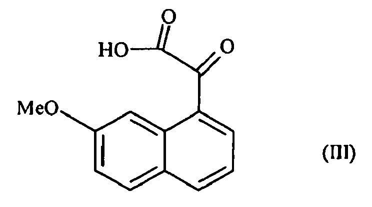 que l'on place dans des conditions d'amination pour conduire au composé de formule (IV) : 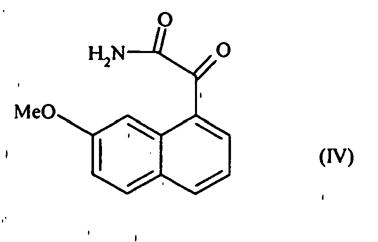 que l'on soumet à l'action d'un système réducteur pour conduire au composé de formule (V): 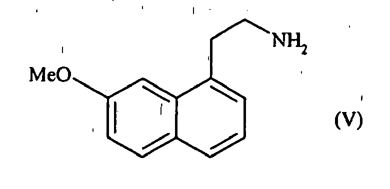 qui est successivement soumis à l'action d'acétate de sodium puis d'anhydride acétique pour conduire au composé de formule (I) que l'on isole sous la forme d'un solide.

2. Procédé de synthèse du composé de formule (I) selon la revendication 1, **caractérisé en ce que** la transformation du composé de formule (II) en composé de formule (III) est réalisée avec NaNH₂.

3. Procédé de synthèse du composé de formule (I) selon la revendication 1, **caractérisé en ce que** la transformation du composé de formule (IV) en composé de formule (V) est réalisée en utilisant le couple BH₃.THF/AlCl₃.

4. Composé de formule (IV) selon la revendication 1 utile en tant qu'intermédiaire de synthèse de l'agomélatine.

5. Utilisation du composé de formule (IV) selon la revendication 4 dans la synthèse de l'agomélatine.

6. Utilisation du composé de formule (II) selon la revendication 1 dans la synthèse de l'agomélatine.

7. Utilisation du composé de formule (III) selon la revendication 1 dans la synthèse de l'agomélatine.

8. Procédé de synthèse de l'agomélatine selon la revendication 1 à partir du composé de formule (III) **caractérisé en ce que** le composé de formule (III) est obtenu selon le procédé de synthèse selon l'une des revendications 1 ou 2.

9. Procédé de synthèse de l'agomélatine selon la revendication 1 à partir du composé de formule (IV) **caractérisé en ce que** le composé de formule (IV) est obtenu selon le procédé de synthèse selon l'une des revendications 1 ou 2.

10. Procédé de synthèse de l'agomélatine selon la revendication 1 à partir du composé de formule (V) **caractérisé en ce que** le composé de formule (V) est obtenu selon le procédé de synthèse selon l'une des revendications 1 à 3.

## Claims

1. Process for the industrial synthesis of the compound of formula (I) 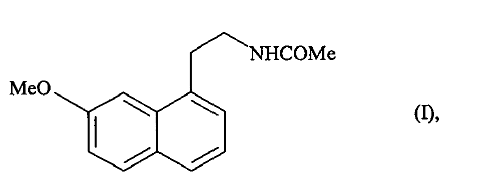 **characterised in that** 3-methoxyacenaphthoquinone of formula (II): 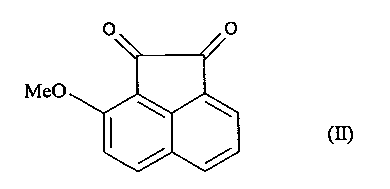 is reacted in the presence of a strong base to yield the compound of formula (III): 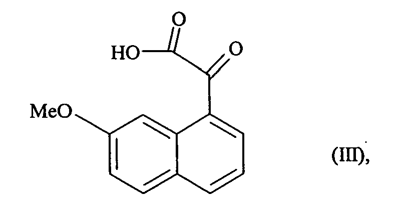 which is subjected to amination conditions to yield the compound of formula (IV): 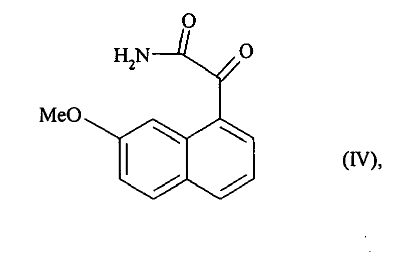 which is subjected to the action of a reducing system to yield the compound of formula (V): 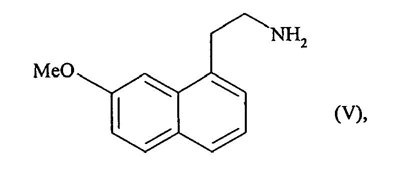 which is successively subjected to the action of sodium acetate and then acetic anhydride to yield the compound of formula (I), which is isolated in the form of a solid.

2. Process for the synthesis of the compound of formula (I) according to claim 1, **characterised in that** the conversion of the compound of formula (II) into the compound of formula (III) is carried out using NaNH₂.

3. Process for the synthesis of the compound of formula (I) according to claim 1, **characterised in that** the conversion of the compound of formula (IV) into the compound of formula (V) is carried out using the couple BH₃.THF/AlCl₃.

4. Compound of formula (IV) according to claim 1 for use as an intermediate in the synthesis of agomelatine.

5. Use of the compound of formula (IV) according to claim 4 in the synthesis of agomelatine.

6. Use of the compound of formula (II) according to claim 1 in the synthesis of agomelatine.

7. Use of the compound of formula (III) according to claim 1 in the synthesis of agomelatine.

8. Process for the synthesis of agomelatine according to claim 1 starting from the compound of formula (III), **characterised in that** the compound of formula (III) is obtained according to the synthesis process according to either claim 1 or claim 2.

9. Process for the synthesis of agomelatine according to claim 1 starting from the compound of formula (IV), **characterised in that** the compound of formula (IV) is obtained according to the synthesis process according to either claim 1 or claim 2.

10. Process for the synthesis of agomelatine according to claim 1 starting from the compound of formula (V), **characterised in that** the compound of formula (V) is obtained according to the synthesis process according to one of claims 1 to 3.

## Patentansprüche

1. Verfahren zur technischen Synthese der Verbindung der Formel (I) 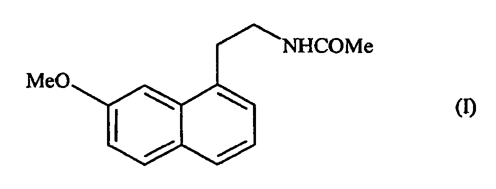 **dadurch gekennzeichnet, dass** man 3-Methoxyacenaphthochinon der Formel (II): 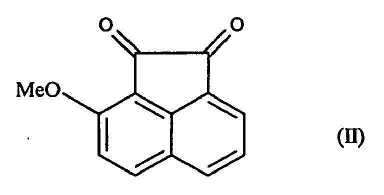 mit einer starken Base umsetzt zur Bildung der Verbindung der Formel (III): 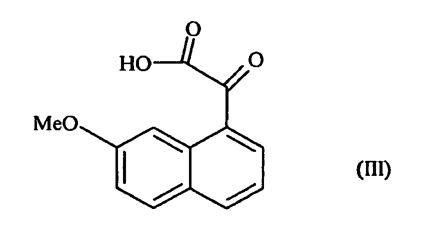 welche man den Bedingungen der Aminierung unterwirft zur Bildung der Verbindung der Formel (IV): 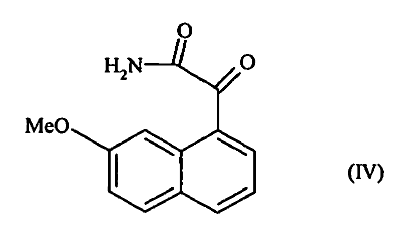 welche man der Einwirkung eines Reduktionssystems unterwirft zur Bildung der Verbindung der Formel (V): 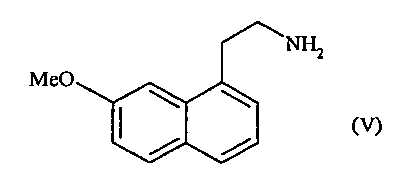 welche nacheinander mit Natriumacetat und dann Essigsäureanhydrid umgesetzt wird zur Bildung der Verbindung der Formel (I), welche man in Form eines Feststoffs isoliert.

2. Verfahren zur Synthese der Verbindung der Formel (I) nach Anspruch 1, **dadurch gekennzeichnet, dass** die Umwandlung der Verbindung der Formel (II) in die Verbindung der Formel (III) mit NaNH₂ bewirkt wird.

3. Verfahren zur Synthese der Verbindung der Formel (I) nach Anspruch 1, **dadurch gekennzeichnet, dass** die Umwandlung der Verbindung der Formel (IV) in die Verbindung der Formel (V) unter Verwendung der Kombination BH_{3˙}THF/AlCl₃ bewirkt wird.

4. Verbindung der Formel (IV) nach Anspruch 1 nützlich als Zwischenprodukt für die Synthese von Agomelatin.

5. Verwendung der Verbindung der Formel (IV) nach Anspruch 4 bei der Synthese von Agomelatin.

6. Verwendung der Verbindung der Formel (II) nach Anspruch 1 bei der Synthese von Agomelatin.

7. Verwendung der Verbindung der Formel (III) nach Anspruch 1 bei der Synthese von Agomelatin.

8. Verfahren zur Synthese von Agomelatin nach Anspruch 1 ausgehend von der Verbindung der Formel (III), **dadurch gekennzeichnet, dass** man die Verbindung der Formel (III) nach dem Syntheseverfahren gemäß einem der Ansprüche 1 oder 2 erhält.

9. Verfahren zur Synthese von Agomelatin nach Anspruch 1 ausgehend von der Verbindung der Formel (IV), **dadurch gekennzeichnet, dass** man die Verbindung der Formel (IV) nach dem Syntheseverfahren gemäß einem der Ansprüche 1 oder 2 erhält.

10. Verfahren zur Synthese von Agomelatin nach Anspruch 1 ausgehend von der Verbindung der Formel (V), **dadurch gekennzeichnet, dass** man die Verbindung der Formel (V) nach dem Syntheseverfahren gemäß einem der Ansprüche 1 bis 3 erhält.
